# EUROPEAN PATENT SPECIFICATION

(11) **EP 2 524 926 B1**
(45) Date of publication and mention of the grant of the patent: **09.03.2016**
(21) Application number: 10843287.3
(22) Date of filing: 27.12.2010
(51) Int. Cl.: C07K 14/465, C12N 15/11, C12N 15/63, A61P 35/00

(54) **ANTICANCER PEPTIDE SEQUENCE**
PEPTIDSEQUENZ GEGEN KREBS
SÉQUENCE DE PEPTIDE ANTICANCÉREUX

(30) Priority: 12.01.2010 KR 20100002910
(43) Date of publication of application: 21.11.2012
(73) Proprietor: SNU R&D FOUNDATION, Seoul 151-818 (KR)
(72) Inventor: BAEK, Sung-Hee, Seoul 151-050 (KR); KIM, Keun ll, Seoul 151-050 (KR); LEE, Ji Min, Seoul 151-058 (KR)
(74) Representative: Bottero, Claudio
(86) International application number: PCT/KR2010/009358
(87) International publication number: WO 2011/087222

(56) References cited:
- EP-A1- 1 864 997
- EP-A2- 2 431 482
- WO-A2-2009/126894
- KR-A- 20050 121 694
- KR-A- 20060 003 903
- KR-A- 20090 092 245
- KR-B1- 100 426 455
- JI MIN LEE ET AL: "ROR[alpha] Attenuates Wnt/[beta]-Catenin Signaling by PKC[alpha]-Dependent Phosphorylation in Colon Cancer", MOLECULAR CELL, vol. 37, no. 2, 28 January 2010 (2010-01-28), pages 183-195, XP055026303, ISSN: 1097-2765, DOI: 10.1016/j.molcel.2009.12.022
- V GIGUERE ET AL: "Isoform-specific amino-terminal domains dictate DNA-binding properties of ROR alpha, a novel family of orphan hormone nuclear receptors.", GENES & DEVELOPMENT, vol. 8, no. 5, 1 March 1994 (1994-03-01), pages 538-553, XP55058864, ISSN: 0890-9369, DOI: 10.1101/gad.8.5.538
- HWANG E J ET AL: "SUMOylation of RORalpha potentiates transcriptional activation function", BIOCHEMICAL AND BIOPHYSICAL RESEARCH COMMUNICATIONS, ACADEMIC PRESS INC. ORLANDO, FL, US, vol. 378, no. 3, 16 January 2009 (2009-01-16), pages 513-517, XP025837853, ISSN: 0006-291X, DOI: 10.1016/J.BBRC.2008.11.072 [retrieved on 2008-11-28]
- JETTEN A M ET AL: "THE ROR NUCLEAR ORPHAN RECEPTOR SUBFAMILY: CRITICAL REGULATORS OF MULTIPLE BIOLOGICAL PROCESSES", PROGRESS IN NUCLEIC ACID RESEARCH AND MOLECULAR BIOLOGY, ACADEMIC PRESS, US, vol. 69, 1 January 2001 (2001-01-01), pages 205-247, XP001068423, ISSN: 0079-6603, DOI: 10.1016/S0079-6603(01)69048-2
- ERIC DUPLUS ET AL: "Phosphorylation and transcriptional activity regulation of retinoid-related orphan receptor alpha 1 by protein kinases C", JOURNAL OF NEUROCHEMISTRY, vol. 104, no. 5, 1 March 2008 (2008-03-01) , pages 1321-1332, XP55059011, ISSN: 0022-3042, DOI: 10.1111/j.1471-4159.2007.05074.x

## Description

### BACKGROUND OF THE INVENTION

### Field of the Invention

The present invention relates to a novel peptide effective in the treatment of colorectal cancer and prostate cancer, a gene encoding the same, and a method of producing the peptide in large amounts.

### Description of the Prior Art

Wnt genes encode a large family of cysteine-rich secreted polypeptides that mediate diverse signaling processes. Aberrant activation of Wnt signaling plays important roles as a major driving leading to developmental defects and tumorigenesis (Klaus, Birchmeier, 2008; Kounek et al., 1997; Morin et al., 1997; Willert et al., 2003). Wnt signaling pathways are divided into two categories: normal Wnt/β-catenin signaling pathway, and abnormal Wnt/Ca²⁺ signaling pathway (Kuhl et al., 2000; Liang et al., 2007; Liu et al., 2005). In the absence of Wnt activity, the level of β-catenin in the cytoplasm remains low due to the degradation of β-catenin by 26S proteasome after paired phosphorylation through casein kinase I (CKI) and glycogen synthase kinase-3β (GSK-3β) (Orford et al., 1997; Salic et al., 2000). The canonical Wnts bind to the Frizzled (Fiz) family proteins and low-density lipoprotein receptor-related (LRP) 5 or 6, and this binding activates disheveled (Dvl) and inhibits the activity of GSK-3β. This inhibition results in the stabilization and subsequent translocation of β-catenin to the nucleus for the regulation of target gene expression with T-cell factor (TCF)/lymphoid enhancer factor (LEF) (Behrens et al., 1996; Giles et al., 2003; Molenaar et al., 1996; Moon et al., 2002).

Non-canonical Wnt signaling pathways affected by Wnt ligands such as Wnt5a have diverse and occasionally opposing roles (Slusarski et al., 1997; Tones et al., 1996). Non-canonical Wnts are both antagonistic and synergistic to canonical Wnt signaling pathway depending on their receptor context. Wnt5a-deficient mice show increased β-catenin signaling in the distal limb, indicating that Wnt5a is involved in the negative regulation of the Wnt/β-catenin signaling pathway (Nemeth et al., 2007). In contrast, Wnt5a has been shown to activate Wnt/β-catenin signaling in the presence of Frz4 and LRP5 (Mikels and Nusse, 2006). Given that the activation of the non-canonical Wnt signaling pathway results in intracellular Ca²⁺ release and activation of Ca²⁺ sensitive enzymes such as Ca²⁺/calmodulin-dependent kinase II (CaMKII) and protein kinase C (PKC), the non-canonical Wnt pathways are apparently different from the canonical Wnt pathway.

### SUMMARY OF THE INVENTION

The present invention provides a novel anticancer peptide.

The present invention provides a gene encoding the novel anticancer peptide.

The present invention provides a method for producing the novel anticancer peptide. Based on the cell culture, colorectal cancer tissue and mouse model studies, the present inventors found that RORα (SEQ ID NO: 1) plays an important role at the point of intersection between the canonical and non-canonical Wnt signaling pathways in attenuating β-catenin transcriptional activity in a phosphorylation-dependent manner in colorectal cancer. As a result of biochemical purification of a RORα-containing complex, it was found that β-catenin is a component of the complex and provides a novel link between RORα and the Wnt signaling pathway. Analysis of RORα interactions with β-catenin revealed that the RORα-mediated inhibition of Wnt/β-catenin signaling requires Wnt5a/PKCα-induced phosphorylation at serine residue 35 of RORα, and the binding of RORα to β-catenin is triggered and enhanced by phosphoiylation of RORα. Interestingly, a reduction in phosphorylation of RORα together with down-regulation of PKCα is associated with the activation of Wnt target genes and the progression of tumors in colorectal cancer tissues. The present inventors found that RORα plays a role in inhibiting the Wnt/β-catenin signaling pathway in a pathophysiological model to regulate cell proliferation and tumor progression. The present inventors developed a method of screening an anticancer agent using RORα, and this method is disclosed in Korean Patent Application No. 10-2009-0001519 (January 8, 2009).

The present invention provides anticancer peptides as defined in the following claims and nucleotide sequences encoding the same.

The present invention also provides a recombinant vector containing a DNA sequence that encodes such anticancer peptides.

The present invention also provides a method of producing an anticancer peptide in large amounts using the recombinant vector containing a DNA sequence that encodes the anticancer peptides as defined above.

### BRIEF DESCRIPTION OF THE DRAWINGS

FIG. 1 shows the results of a cell migration assay for HCT116 cells which express RORα, RORαS35A or RORαS35D.
FIG. 2 shows the inhibition of anchorage-independent growth of Wnt5a-treated HCT116 cells which express RORα, RORαS35A or RORαS35D.
FIG. 3 shows the comparison of the ratio of phosphorylated RORα to pan-RORα between 30 human colorectal cancer tissues and normal tissues.
FIG. 4 shows the comparison of the ratio of phosphorylated PKCα to Pan-PKCα between 30 human colorectal cancer tissues and normal tissues.
FIG. 5 shows the inhibition of prostate cancer by the anticancer peptide RORα stably expressed in PC cells. In FIG. 5, (A): MOCK; (B): a nude mouse injected with PC cells having RORα stably expressed therein; (C): a prostate separated from MOCK (left) andaprostate separated from the nude mouse injected with PC cells having RORα stably expressed therein.
FIG. 6 shows the anticancer effects of RORα (SEQ ID NO: 3) and RORα (SEQ ID NO: 4) stably expressed in LNCap cells. In FIG. 6, (A): MOCK, (B): a nude mouse injected with LNCap cells having RORα1 (SEQ ID NO: 4) stably expressed therein; (C): a nude mouse injected with LNCap cells having RORα4 (SEQ ID NO: 5) stably expressed therein; (D), (E) and (F): cancer masses extracted from LNCap Mock, LNCaP/RORα1 and LNCaP/RORα4 at 10 weeks after injection of the cells.

### DETAILED DESCRIPTION OF THE INVENTION

The present invention provides anticancer peptides as defined in claims 1-4, which are effective in the treatment of prostate cancer, and a gene encoding the same. The present invention also provides a method for producing the anticancer peptide. The production method is preferably performed by a genetic engineering method, but is not limited.

In one aspect, the present invention provides an anticancer peptide consisting of amino acid residues 31 to 40 of SEQ ID NO: 1 and having a phosphorylated serine at position 35 of SEQ ID NO:1, or a functional equivalent thereof.

As used herein, the term "functional equivalent" is intended to include all amino acid sequence variants having amino acid substitutions in some or all of the anticancer peptide of SEQ ID NO: 1 or amino acid additions or deletions in some of the anticancer peptide and having substantially the same physiological activity as the anticancer peptide of SEQ ID NO:1. The amino acid substitutions are preferably conservative substitutions. Examples of the conservative substitutions of naturally occurring amino acids include aliphatic amino acids (Gly, Ala, Pro), hydrophobic amino acids (Ile, Leu, Val), aromatic amino acids (Phe, Tyr, Trp), acidic amino acids (Asp, Glu), basic amino acids (His, Lys, Arg, Gln, Asn) and sulfur-containing amino acids (Cys, Met). Deletions of the amino acids are preferably located at portions that are not involved directly in the physiological activity of the anticancer peptide.

The anticancer peptide of the present invention may be a portion of a peptide consisting of the entire sequence of SEQ ID NO: 1.

According to the invention, the functional equivalent of the anticancer peptide of the present invention is either a peptide of SEQ ID NO 2 having a serine-to-aspartic acid substitution at position 35 of SEQ ID NO: 1 or a peptide of SEQ NO: 3 having a serine-to-glutamic acid substitution at position 35 of SEQ ID NO:1. The aspartic acid and glutamic acid substitutions have properties similar to phosphorylated serine, and thus exhibit effective anticancer activity.

The functional equivalent may be a portion of a peptide consisting of the entire sequence of SEQ ID NO: 2 or SEQ ID NO: 3. In one aspect of the invention, the functional equivalent is a peptide consisting of amino acid residues 31 to 40 of SEQ ID NO: 2,, a peptide consisting of amino acid residues 31 to 40 of SEQ ID NO: 3, or a peptide comprising amino acid residues 31 to 40 of SEQ ID NO: 3.

In a second aspect, the present invention a nucleotide sequence, which encodes the anticancer peptide according to claims 1, 3 and 4, and nucleotide sequences equivalent thereto.

As used herein, "nucleotide sequences equivalent" include the codon degenerate sequence of the anticancer peptide.

As used herein, the term "codon degenerate sequence" means a nucleotide sequence which differs from the naturally occurring sequence, but encodes a peptide having the same sequence as that of the anticancer peptide disclosed in the present invention.

In a third aspect, the present invention provides a recombinant vector comprising a DNA fragment which encodes any one of the anticancer peptides of claims 1-4 or functional equivalents thereof.

As used herein, the term "vector" means a nucleic acid molecule that can carry another nucleic acid bound thereto. As used herein, the term "expression vector" is intended to include a plasmid, cosmid or phage, which can be used to synthesize a protein encoded by a recombinant gene carried by said vector. A preferred vector is a vector that can self-replicate and express a nucleic acid bound thereto.

In a fourth aspect, the present invention provides a cell transformed with a recombinant vector as defined above.

As used herein, the term "transformation" means that foreign DNA or RNA is absorbed into a cell to change the genotype of the cell.

Cells suitable for transformation include, but are not limited to, prokaryotic cells, fungal cells, plant cells and animal cells.

In a fifth aspect, the present invention provides a method of producing an anticancer peptide using a cell transformed with a recombinant vector as defined above.

Hereinafter, the present invention will be described in further detail with reference to examples. It is to be understood, however, that these examples are for illustrative purposes only and are not intended to limit the scope of the present invention.

### Example 1: Reduction in cell migration by RORαS35D (SEQ ID NO: 2)

HCT116 cells which stably express RORα (SEQ ID NO: 1), RORαS35A or RORαS35D (SEQ ID NO: 2) were used in a transwell cell migration assay. The transwell cell migration assay was carried out according to the method of Kim et al. (Roles of SUMOylation of a reptin chromatin remodeling complex in cancer metastasis," Nat. Cell Biol. 8, 631-639). Cultured cells were pretreated with Wnt5a protein (100 ng/ml) for 2 hours, and 2.5 x 10⁴ HCT 116 cells were placed on a 24-well transwell chamber assay plate (BD Biocoat, BD Biosciences). 15% fetal bovine serum-containing McCoy's 5A medium as a chemoattractant was added below the chamber. After 22 hours of incubation, the cells that migrated to the chamber below the filter were fixed with 100% methanol, stained with DAPI, and quantified by counting the total number of cells in four different fields. All the experiments were performed according to the manufacturer's protocol. Values are expressed as means ± standard deviations for at least three independent experiments.

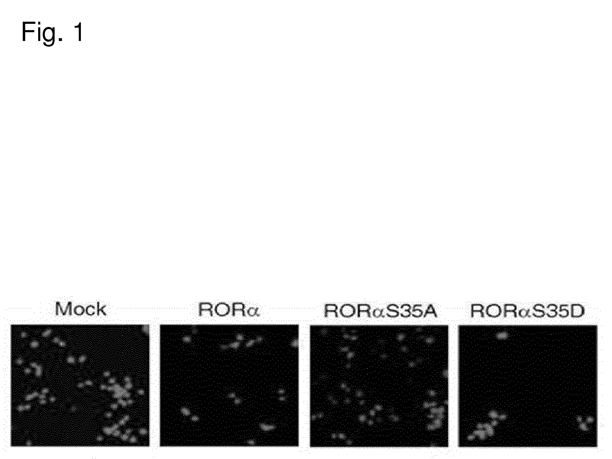

In the cell migration assay, treatment with Wnt5a reduced the migration of HCT116 colorectal cancer cells compared to non-treated cells, and the migration of the RORαS3 5-expressing HCT116 colorectal cancer cells was significantly reduced (FIG. 1).

### Example 2: Reduction in colony-forming ability by RORαS35D (SEQ ID NO: 2)

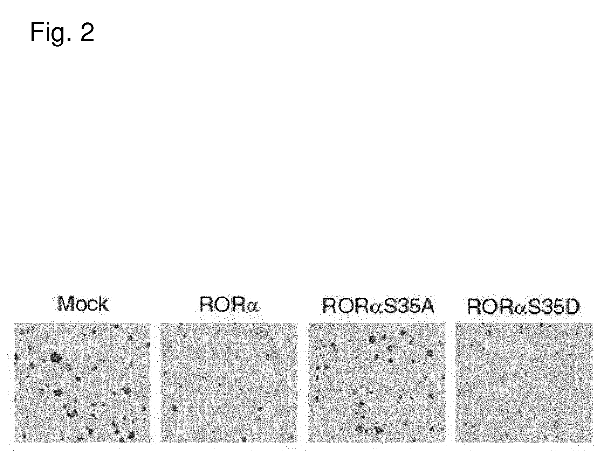

In order to measure anchorage-independent growth, the growth of HCT116 cells which stably express RORα (SEQ ID NO:1), RORαS35A or RORαS35D (SEQ ID NO: 2) was analyzed on semisolid medium. Specifically, 10⁵ cells were placed on McCoy's 5A medium containing 0.4% noble agar and 10% FBS. The cells were incubated in 5% CO₂ for 3 weeks, and the formation of colonies containing more than 50 cells was analyzed. As a result, the anchorage-independent growth of the RORαS35D-expressing cells was significantly inhibited compared to a control group (FIG. 2).

### Example 3: Reduction in phosphorylation of RORα in human colorectal cancer

In order to confirm the clinical significance of RORα, the expressions of RORα in 30 human colorectal cancer tissues were examined and compared to those in normal mucous samples. In an immunoassay for anti-phospho-RORα S35D IgG, it was seen that RORα phosphorylation was reduced in 22 of 30 colorectal cancer tissues (>70%), whereas there was little or no change in the expression of pan-RORα (Table 1).

**Table 1: Immunological assay for anti-phospho-ROR in colorectal cancer tissues and normal mucous sample**

| | phospho-RORαS35 | | pan-RORα | | phospho/pan-RORα | |
|---|---|---|---|---|---|---|
| No. | Normal | Tumor | Normal | Tumor | Normal | Tumor |
| 1 | 13.57 | 14.74 | 82.27 | 83.43 | 0.1649 | 0.1767 |
| 2 | 7.37 | 8.75 | 65.72 | 78.45 | 0.1121 | 0.1115 |
| 3 | 4.05 | 3.02 | 66.22 | 49.42 | 0.0612 | 0.0611 |
| 4 | 59.22 | 7.68 | 128.94 | 100.10 | 0.4593 | 0.0767 |
| 5 | 20.86 | 2.93 | 56.03 | 70.64 | 0.3723 | 0.0415 |
| 6 | 38.53 | 1.38 | 83.76 | 86.27 | 0.4600 | 0.0160 |
| 7 | 19.97 | 1.30 | 74.14 | 84.14 | 0.2654 | 0.0155 |
| 8 | 8.83 | 8.52 | 85.61 | 83.76 | 0.1031 | 0.1017 |
| 9 | 11.65 | 0.69 | 58.82 | 84.46 | 0.1981 | 0.0082 |
| 10 | 12.59 | 0.32 | 83.98 | 74.36 | 0.1499 | 0.0043 |
| 11 | 29.65 | 0.09 | 76.82 | 102.68 | 0.3860 | 0.0009 |
| 12 | 5.21 | 4.19 | 90.59 | 73.12 | 0.0575 | 0.0573 |
| 13 | 33.98 | 15.34 | 89:36 | 93.76 | 0.3803 | 0.1636 |
| 14 | 43.75 | 7.74 | 99.04 | 99.81 | 0.4417 | 0.0775 |
| 15 | 5.12 | 5.07 | 86.61 | 84.67 | 0.0591 | 0.0599 |
| 16 | 24.74 | 4.37 | 84.46 | 69.23 | 0.2929 | 0.0631 |
| 17 | 12.05 | 1.51 | 94.36 | 98.01 | 0.1277 | 0,0154 |
| 18 | 38.84 | 9.03 | 85.65 | 87.52 | 0.4535 | 0.1032 |
| 19 | 10.78 | 3.56 | 77.58 | 66.82 | 0.1390 | 0.0533 |
| 20 | 26.59 | 24.88 | 98.26 | 89.21 | 0.2706 | 0.2789 |
| 21 | 10.41 | 17.38 | 81.78 | 85.73 | 0.1273 | 0.2027 |
| 22 | 45.71 | 12.38 | 92.06 | 68.92 | 0.4965 | 0.1796 |
| 23 | 30.08 | 3.69 | 93.84 | 84.05 | 0.3205 | 0.0439 |
| 24 | 35.70 | 1.69 | 79.64 | 88.45 | 0.4483 | 0.0191 |
| 25 | 33.39 | 10.17 | 93.99 | 94.16 | 0.3553 | 0.1080 |
| 26 | 35.33 | 9.86 | 96.75 | 96.96 | 0.3652 | 0.1017 |
| 27 | 30.92 | 0.94 | 95.62 | 100.75 | 0.3234 | 0.0093 |
| 28 | 20.45 | 0.48 | 90.98 | 97.66 | 0.2248 | 0.0049 |
| 29 | 12.42 | 0.08 | 85.30 | 94.22 | 0.1456 | 0.0008 |
| 30 | 16.03 | 1.21 | 89.73 | 98.67 | 0.1786 | 0.0123 |

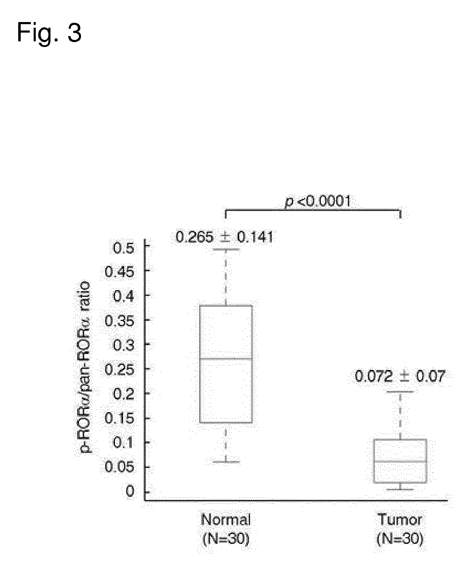

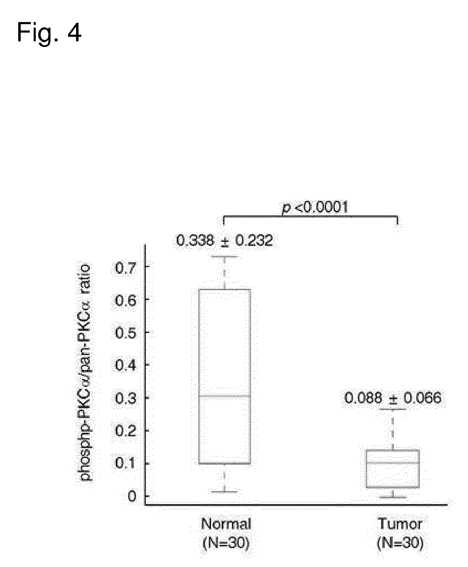

Statistical analysis showed that phosphorylated RORα and phosphorylated PKCα were significantly reduced in the cancer tissue samples compared to the normal tissues (FIGS. 3 and 4). These data indicate that the phosphorylation of RORα is frequent in human colorectal cancer.

### Example 4: Inhibition of growth of prostate cancer cells using the PC3 cell line that stably expresses RORα

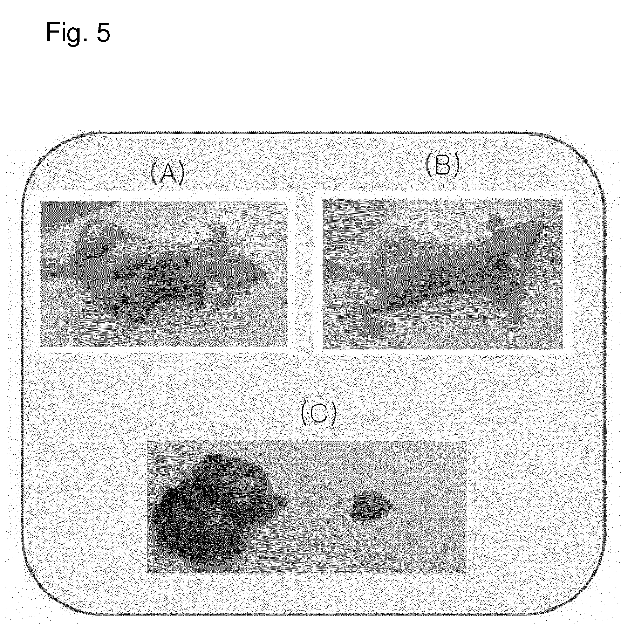

PC3 is a prostate tissue cell line in which cancer is activated to the metastatic process. PC3 cells are available from ATCC (ATCC^{®} Number: CRL-1435™). Using this cell line, a stable cell line was constructed such that it can stably express the RORα protein. Specifically, an expression plasmid having a DNA sequence which can be expressed as the RORα protein was constructed using a p3XFlag-CMV10 vector (produced by Sigma-Aldrich) and transfected into cells. Then, cells having the RORα DNA sequence stably transfected in the intracellular chromosome were selected using the antibiotic present in the plasmid, thereby constructing a cell line that constitutively expresses RORα. PC3 has high cancer activity, and thus when it is subcutaneously injected into nude mice (Orient Bio Inc.), cancer is easily formed as shown in a PC3 mock (FIGS. 5(A) and 5(C)). However, in the case of the stable cell line (FIG. 5(B) and 5(C)) that stably expresses RORα, little or no cancer formation occurs due to the function of the expressed RORα protein. FIG. 5(C) shows a cancer mass extracted 4 weeks after injection of the cell line (left: MOCK; right: cell line stably expressed in RORα cells).

### Example 5: Inhibition of growth of prostate cancer cells using the LNCaP cell line that stably expresses RORα

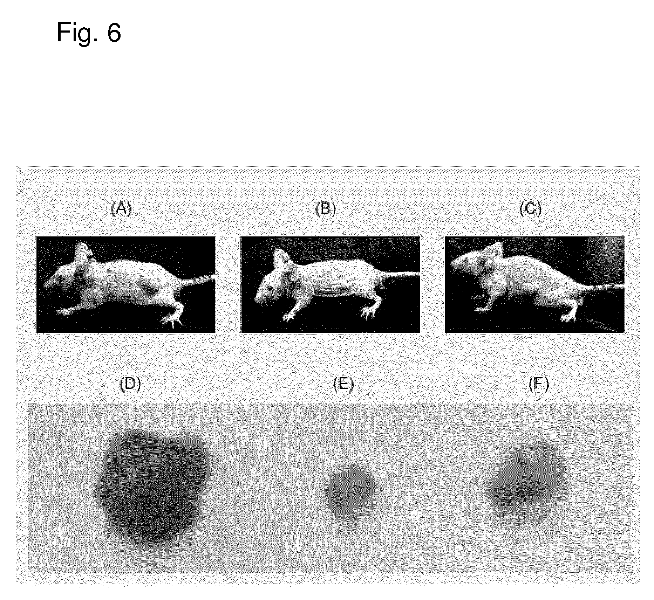

Like PC3, LNCaP is also a prostate tissue cell line in which cancer is activated to the metastatic process. LNCaP cells are available from ATCC (ATCC® Number: CRL-1740™). Using this cell line, stable cell lines were constructed such that they can stably express proteins of RORα1 (SEQ ID NO: 4) and RORα4 (SEQ ID NO: 5), respectively. RORα4 is a protein of a RORα1 isotype family, which does not contain a peptide sequence important in a cancer inhibition process, unlike RORα1. Expression plasmids having DNA sequences that can be expressed as RORα1 and RORα4, respectively, were constructed using a p3XFlag-CMV10 vector (produced by Sigma-Aldrich) and transfected into cells. Then, cells having each of the RORα1 and RORα4 DNA sequences stably transfected in the intracellular chromosome were selected using the antibiotic present in the plasmid, thereby constructing cell lines that constitutively express RORα. Like PC3, LNCaP also has high cancer activity, and thus when it is subcutaneously injected into 6-week old nude mice (Orient Bio Inc.), cancer is easily formed as shown in a PC3 mock (FIGS. 6(A) and 6(C)). However, in the case of the cell line LNCaP/RORα1 that stably expresses RORα1, little or no cancer formation occurred due to the function of RORα1 having the peptide sequence functioning to inhibit expressed cancer (FIG. 6(B) and 6(E)). Unlike this cell line, in the case of the stable cell line LNCaP/RORα4 that stably expresses RORα4 whose N-terminal end differs from that of RORα1 and which has no serine for phosphorylation at position 35, the inhibition of cancer formation was not substantially observed (FIGS. 6(C) and 6 (F). FIGS. 6(D), 6(E) and 6(F) show cancer masses extracted from LNCap mock, LNCaP/RORα1 and LNCaP/RORα4, respectively, 10 weeks after injection of the cell lines.

As described above, the anticancer peptide according to the present invention is RORα having a phosphorylated serine at position 35 of SEQ ID NO: 2, or a functional equivalent, and specifically inhibits β-catenin transcriptional activity. Thus, the anticancer peptide can be effectively used for the prevention and treatment of cancer mediated by the Wnt/β-catenin signaling pathway. Particularly, the anticancer peptide can be effectively used for colorectal cancer and prostate cancer.
## Claims

1. An anticancer peptide, which comprises amino acid residues 31 to 40 of SEQ ID NO: 3.

2. An anticancer peptide consisting of amino acid residues 31 to 40 of SEQ ID NO: 1 and having a phosphorylated serine at position 35 of SEQ ID NO: 1.

3. An anticancer peptide consisting of amino acid residues 31 to 40 of SEQ ID NO: 2.

4. An anticancer peptide consisting of amino acid residues 31 to 40 of SEQ NO: 3.

5. A nucleotide sequence encoding an anticancer peptide of any one of claims 1, 3 and 4.

6. A recombinant vector comprising a DNA fragment encoding an anticancer peptide of any one of claims 1-4.

7. A cell transformed with the recombinant vector of claim 6.

8. A method for producing an anticancer peptide, the method comprising culturing the transformed cell of claim 7 and isolating an anticancer peptide of anyone of claims 1-4 from the cultured cell.

## Patentansprüche

1. Peptid zur Antikrebstherapie, das Aminosäurenreste 31 bis 40 von SEQ ID NR: 3 umfasst.

2. Peptid zur Antikrebstherapie, bestehend aus Aminosäurenresten 31 bis 40 von SEQ ID NR:1 und umfassend ein phosphoryliertes Serin an Position 35 von SEQ ID NR: 1.

3. Peptid zur Antikrebstherapie, bestehend aus Aminosäurenresten 31 bis 40 von SEQ ID NR:2.

4. Peptid zur Antikrebstherapie, bestehend aus Aminosäurenresten 31 bis 40 von SEQ ID NR:3.

5. Nukleotidsequenz, kodierend ein Peptid zur Antikrebstherapie gemäß einem der Ansprüche 1, 3 und 4.

6. Rekombinanter Vektor umfassend ein DNA-Fragment, kodierend ein Peptid zur Antikrebstherapie gemäß einem der Ansprüche 1 bis 4.

7. Zelle, die mit dem rekombinanten Vektor gemäß Anspruch 6 transformiert ist.

8. Verfahren zur Herstellung eines Peptids zur Antikrebstherapie, wobei das Verfahren das Kultivieren der transformierten Zelle gemäß Anspruch 7 und Isolieren eines Peptids zur Antikrebstherapie gemäß einem der Ansprüche 1 bis 4 aus der kultivierten Zelle umfasst.

## Revendications

1. Peptide anticancéreux qui comprend des résidus d'acides aminés 31 à 40 de SEQ ID N° 3.

2. Peptide anticancéreux composé de résidus d'acides aminés 31 à 40 de SEQ ID N° 1 et ayant une sérine phosphorylée en position 35 de SEQ ID N° 1.

3. Peptide anticancéreux composé de résidus d'acides aminés 31 à 40 de SEQ ID N° 2.

4. Peptide anticancéreux composé de résidus d'acides aminés 31 à 40 de SEQ ID N° 3.

5. Séquence nucléotidique codant un peptide anticancéreux de l'une quelconque des revendications 1, 3 et 4.

6. Vecteur recombinant comprenant un fragment d'ADN codant un peptide anticancéreux de l'une quelconque des revendications 1 à 4.

7. Cellule transformée avec le vecteur recombinant de la revendication 6.

8. Procédé de production d'un peptide anticancéreux, le procédé comprenant la culture de la cellule transformée de la revendication 7 et l'isolement d'un peptide anticancéreux de l'une quelconque des revendications 1 à 4 à partir de la cellule transformée.


<110> Seoul Nation University Industry Foundation
<120> Anti-cancer peptide sequences
<160> 5
<170> KopatentIn 1.71
<210> 1
   <211> 523
   <212> PRT
   <213> Homo sapiens
<400> 1
<210> 2
   <211> 523
   <212> PRT
   <213> Artificial Sequence
<220>
   <223> Modified RORalpha Proten in phosphorylation site from serine to Aspartate
<400> 2
   Met Glu Ser Ala Pro Ala Ala Pro Asp Pro Ala Ala Ser Glu Pro Gly
    1               5                  10                  15
   Ser Ser Gly Ala Asp Ala Ala Ala Gly Ser Arg Glu Thr Pro Leu Asn
               20                  25                  30
   Gln Glu Asp Ala Arg Lys Ser Glu Pro Pro Ala Pro Val Arg Arg Gln
           35                  40                  45
   Ser Tyr Ser Ser Thr Ser Arg Gly Ile Ser Val Thr Lys Lys Thr His
       50                  55                  60
   Thr Ser Gln Ile Glu Ile Ile Pro Cys Lys Ile Cys Gly Asp Lys Ser
   65                  70                  75                  80
   Ser Gly Ile His Tyr Gly Val Ile Thr Cys Glu Gly Cys Lys Gly Phe
                   85                  90                  95
   Phe Arg Arg Ser Gln Gln Ser Asn Ala Thr Tyr Ser Cys Pro Arg Gln
               100                 105                 110
   Lys Asn Cys Leu Ile Asp Arg Thr Ser Arg Asn Arg Cys Gln His Cys
           115                 120                 125
   Arg Leu Gln Lys Cys Leu Ala Val Gly Met Ser Arg Asp Ala Val Lys
       130                 135                 140
   Phe Gly Arg Met Ser Lys Lys Gln Arg Asp Ser Leu Tyr Ala Glu Val
   145                 150                 155                 160
   Gln Lys His Arg Met Gln Gln Gln Gln Arg Asp His Gln Gln Gln Pro
                   165                 170                 175
   Gly Glu Ala Glu Pro Leu Thr Pro Thr Tyr Asn Ile Ser Ala Asn Gly
               180                 185                 190
   Leu Thr Glu Leu His Asp Asp Leu Ser Asn Tyr Ile Asp Gly His Thr
           195                 200                 205
   Pro Glu Gly Ser Lys Ala Asp Ser Ala Val Ser Ser Phe Tyr Leu Asp
       210                 215                 220
   Ile Gln Pro Ser Pro Asp Gln Ser Gly Leu Asp Ile Asn Gly Ile Lys
   225                 230                 235                 240
   Pro Glu Pro Ile Cys Asp Tyr Thr Pro Ala Ser Gly Phe Phe Pro Tyr
                   245                 250                 255
   Cys Ser Phe Thr Asn Gly Glu Thr Ser Pro Thr Val Ser Met Ala Glu
               260                 265                 270
   Leu Glu His Leu Ala Gln Asn Ile Ser Lys Ser His Leu Glu Thr Cys
           275                 280                 285
   Gln Tyr Leu Arg Glu Glu Leu Gln Gln Ile Thr Trp Gln Thr Phe Leu
       290                 295                 300
   Gln Glu Glu Ile Glu Asn Tyr Gln Asn Lys Gln Arg Glu Val Met Trp
   305                 310                 315                 320
   Gln Leu Cys Ala Ile Lys Ile Thr Glu Ala Ile Gln Tyr Val Val Glu
                   325                 330                 335
   Phe Ala Lys Arg Ile Asp Gly Phe Met Glu Leu Cys Gln Asn Asp Gln
               340                 345                 350
   Ile Val Leu Leu Lys Ala Gly Ser Leu Glu Val Val Phe Ile Arg Met
           355                 360                 365
   Cys Arg Ala Phe Asp Ser Gln Asn Asn Thr Val Tyr Phe Asp Gly Lys
       370                 375                 380
   Tyr Ala Ser Pro Asp Val Phe Lys Ser Leu Gly Cys Glu Asp Phe Ile
   385                 390                 395                 400
   Ser Phe Val Phe Glu Phe Gly Lys Ser Leu Cys Ser Met His Leu Thr
                   405                 410                 415
   Glu Asp Glu Ile Ala Leu Phe Ser Ala Phe Val Leu Met Ser Ala Asp
               420                 425                 430
   Arg Ser Trp Leu Gln Glu Lys Val Lys Ile Glu Lys Leu Gln Gln Lys
           435                 440                 445
   Ile Gln Leu Ala Leu Gln His Val Leu Gln Lys Asn His Arg Glu Asp
       450                 455                 460
   Gly Ile Leu Thr Lys Leu Ile Cys Lys Val Ser Thr Leu Arg Ala Leu
   465                 470                 475                 480
   Cys Gly Arg His Thr Glu Lys Leu Met Ala Phe Lys Ala Ile Tyr Pro
                   485                 490                 495
   Asp Ile Val Arg Leu His Phe Pro Pro Leu Tyr Lys Glu Leu Phe Thr
               500                 505                 510
   Ser Glu Phe Glu Pro Ala Met Gln Ile Asp Gly
           515                 520
<210> 3
   <211> 523
   <212> PRT
   <213> Artificial Sequence
<220>
   <223> Mutated ROR alpha (35 serine to Glutamate)
<400> 3
   Met Glu Ser Ala Pro Ala Ala Pro Asp Pro Ala Ala Ser Glu Pro Gly
    1               5                  10                  15
   Ser Ser Gly Ala Asp Ala Ala Ala Gly Ser Arg Glu Thr Pro Leu Asn
               20                  25                  30
   Gln Glu Glu Ala Arg Lys Ser Glu Pro Pro Ala Pro Val Arg Arg Gln
           35                  40                  45
   Ser Tyr Ser Ser Thr Ser Arg Gly Ile Ser Val Thr Lys Lys Thr His
       50                  55                  60
   Thr Ser Gln Ile Glu Ile Ile Pro Cys Lys Ile Cys Gly Asp Lys Ser
   65                  70                  75                  80
   Ser Gly Ile His Tyr Gly Val Ile Thr Cys Glu Gly Cys Lys Gly Phe
                   85                  90                  95
   Phe Arg Arg Ser Gln Gln Ser Asn Ala Thr Tyr Ser Cys Pro Arg Gln
               100                 105                 110
   Lys Asn Cys Leu Ile Asp Arg Thr Ser Arg Asn Arg Cys Gln His Cys
           115                 120                 125
   Arg Leu Gln Lys Cys Leu Ala Val Gly Met Ser Arg Asp Ala Val Lys
       130                 135                 140
   Phe Gly Arg Met Ser Lys Lys Gln Arg Asp Ser Leu Tyr Ala Glu Val
   145                 150                 155                 160
   Gln Lys His Arg Met Gln Gln Gln Gln Arg Asp His Gln Gln Gln Pro
                   165                 170                 175
   Gly Glu Ala Glu Pro Leu Thr Pro Thr Tyr Asn Ile Ser Ala Asn Gly
               180                 185                 190
   Leu Thr Glu Leu His Asp Asp Leu Ser Asn Tyr Ile Asp Gly His Thr
           195                 200                 205
   Pro Glu Gly Ser Lys Ala Asp Ser Ala Val Ser Ser Phe Tyr Leu Asp
       210                 215                 220
   Ile Gln Pro Ser Pro Asp Gln Ser Gly Leu Asp Ile Asn Gly Ile Lys
   225                 230                 235                 240
   Pro Glu Pro Ile Cys Asp Tyr Thr Pro Ala Ser Gly Phe Phe Pro Tyr
                   245                 250                 255
   Cys Ser Phe Thr Asn Gly Glu Thr Ser Pro Thr Val Ser Met Ala Glu
               260                 265                 270
   Leu Glu His Leu Ala Gln Asn Ile Ser Lys Ser His Leu Glu Thr Cys
           275                 280                 285
   Gln Tyr Leu Arg Glu Glu Leu Gln Gln Ile Thr Trp Gln Thr Phe Leu
       290                 295                 300
   Gln Glu Glu Ile Glu Asn Tyr Gln Asn Lys Gln Arg Glu Val Met Trp
   305                 310                 315                 320
   Gln Leu Cys Ala Ile Lys Ile Thr Glu Ala Ile Gln Tyr Val Val Glu
                   325                 330                 335
   Phe Ala Lys Arg Ile Asp Gly Phe Met Glu Leu Cys Gln Asn Asp Gln
               340                 345                 350
   Ile Val Leu Leu Lys Ala Gly Ser Leu Glu Val Val Phe Ile Arg Met
           355                 360                 365
   Cys Arg Ala Phe Asp Ser Gln Asn Asn Thr Val Tyr Phe Asp Gly Lys
       370                 375                 380
   Tyr Ala Ser Pro Asp Val Phe Lys Ser Leu Gly Cys Glu Asp Phe Ile
   385                 390                 395                 400
   Ser Phe Val Phe Glu Phe Gly Lys Ser Leu Cys Ser Met His Leu Thr
                   405                 410                 415
   Glu Asp Glu Ile Ala Leu Phe Ser Ala Phe Val Leu Met Ser Ala Asp
               420                 425                 430
   Arg Ser Trp Leu Gln Glu Lys Val Lys Ile Glu Lys Leu Gln Gln Lys
           435                 440                 445
   Ile Gln Leu Ala Leu Gln His Val Leu Gln Lys Asn His Arg Glu Asp
       450                 455                 460
   Gly Ile Leu Thr Lys Leu Ile Cys Lys Val Ser Thr Leu Arg Ala Leu
   465                 470                 475                 480
   Cys Gly Arg His Thr Glu Lys Leu Met Ala Phe Lys Ala Ile Tyr Pro
                   485                 490                 495
   Asp Ile Val Arg Leu His Phe Pro Pro Leu Tyr Lys Glu Leu Phe Thr
               500                 505                 510
   Ser Glu Phe Glu Pro Ala Met Gln Ile Asp Gly
           515                 520
<210> 4
   <211> 523
   <212> PRT
   <213> Homo sapiens
<400> 4
<210> 5
   <211> 466
   <212> PRT
   <213> Homo sapiens
<400> 5
   Met Met Tyr Phe Val Ile Met Lys Ala Gln Ile Glu Ile Ile Pro Cys
   1               5                   10                  15
   Lys Ile Cys Gly Asp Lys Ser Ser Gly Ile His Tyr Gly Val Ile Thr
               20                  25                  30
   Cys Glu Gly Cys Lys Gly Phe Phe Arg Arg Ser Gln Gln Ser Asn Ala
           35                  40                  45
   Thr Tyr Ser Cys Pro Arg Gln Lys Asn Cys Leu Ile Asp Arg Thr Ser
        50                  55                  60
   Arg Asn Arg Cys Gln His Cys Arg Leu Gln Lys Cys Leu Ala Val Gly
    65                  70                  75                  80
   Met Ser Arg Asp Ala Val Lys Phe Gly Arg Met Ser Lys Lys Gln Arg
                    85                  90                  95
   Asp Ser Leu Tyr Ala Glu Val Gln Lys His Arg Met Gln Gln Gln Gln
               100                 105                 110
   Arg Asp His Gln Gln Gln Pro Gly Glu Ala Glu Pro Leu Thr Pro Thr
           115                 120                 125
   Tyr Asn Ile Ser Ala Asn Gly Leu Thr Glu Leu His Asp Asp Leu Ser
       130                 135                 140
   Thr Tyr Met Asp Gly His Thr Pro Glu Gly Ser Lys Ala Asp Ser Ala
   145                 150                 155                 160
   Val Ser Ser Phe Tyr Leu Asp Ile Gln Pro Ser Pro Asp Gln Ser Gly
                   165                 170                 175
   Leu Asp Ile Asn Gly Ile Lys Pro Glu Pro Ile Cys Asp Tyr Thr Pro
               180                 185                 190
   Ala Ser Gly Phe Phe Pro Tyr Cys Ser Phe Thr Asn Gly Glu Thr Ser
           195                 200                 205
   Pro Thr Val Ser Met Ala Glu Leu Glu His Leu Ala Gln Asn Ile Ser
       210                 215                 220
   Lys Ser His Leu Glu Thr Cys Gln Tyr Leu Arg Glu Glu Leu Gln Gln
   225                 230                 235                 240
   Ile Thr Trp Gln Thr Phe Leu Gln Glu Glu Ile Glu Asn Tyr Gln Asn
                   245                 250                 255
   Lys Gln Arg Glu Val Met Trp Gln Leu Cys Ala Ile Lys Ile Thr Glu
               260                 265                 270
   Ala Ile Gln Tyr Val Val Glu Phe Ala Lys Arg Ile Asp Gly Phe Met
           275                 280                 285
   Glu Leu Cys Gln Asn Asp Gln Ile Val Leu Leu Lys Ala Gly Ser Leu
       290                 295                 300
   Glu Val Val Phe Ile Arg Met Cys Arg Ala Phe Asp Ser Gln Asn Asn
   305                 310                 315                 320
   Thr Val Tyr Phe Asp Gly Lys Tyr Ala Ser Pro Asp Val Phe Lys Ser
                   325                 330                 335
   Leu Gly Cys Glu Asp Phe Ile Ser Phe Val Phe Glu Phe Gly Lys Ser
               340                 345                 350
   Leu Cys Ser Met His Leu Thr Glu Asp Glu Ile Ala Leu Phe Ser Ala
           355                 360                 365
   Phe Val Leu Met Ser Ala Asp Arg Ser Trp Leu Gln Glu Lys Val Lys
       370                 375                 380
   Ile Glu Lys Leu Gln Gln Lys Ile Gln Leu Ala Leu Gln His Val Leu
   385                 390                 395                 400
   Gln Lys Asn His Arg Glu Asp Gly Ile Leu Thr Lys Leu Ile Cys Lys
                   405                 410                 415
   Val Ser Thr Leu Arg Ala Leu Cys Gly Arg His Thr Glu Lys Leu Met
               420                 425                 430
   Ala Phe Lys Ala Ile Tyr Pro Asp Ile Val Arg Leu His Phe Pro Pro
           435                 440                 445
   Leu Tyr Lys Glu Leu Phe Thr Ser Glu Phe Glu Pro Ala Met Gln Ile
       450                 455                 460
   Asp Gly
   465